# EUROPEAN PATENT SPECIFICATION

(11) **EP 3 976 165 B1**
(45) Date of publication and mention of the grant of the patent: **26.11.2025**
(21) Application number: 20818750.0
(22) Date of filing: 01.05.2020
(51) Int. Cl.: A61N 1/05, A61N 1/36

(54) **DEVICES FOR TREATING TINNITUS USING ELECTRICAL STIMULATION**
VORRICHTUNGEN ZUR BEHANDLUNG VON TINNITUS MITTELS ELEKTRISCHER STIMULATION
DISPOSITIFS DE TRAITEMENT DES ACOUPHÈNES À L'AIDE D'UNE STIMULATION ÉLECTRIQUE

(30) Priority: 03.06.2019 US 201962856535 P
(43) Date of publication of application: 06.04.2022
(73) Proprietor: Mayo Foundation for Medical Education and Research, Rochester, MN 55905 (US)
(72) Inventor: CARLSON, Matthew L., Rochester, Minnesota 55902-1556 (US)
(74) Representative: Grünecker Patent- und Rechtsanwälte PartG mbB
(86) International application number: PCT/US2020/031020
(87) International publication number: WO 2020/247125

(56) References cited:
- WO-A1-2017/213978
- WO-A1-2017/213978
- US-A1- 2007 021 804
- US-A1- 2010 305 676
- US-A1- 2013 281 812
- US-A1- 2014 163 692
- US-A1- 2017 050 027
- US-A1- 2019 167 985
- US-B1- 8 880 193

## Description

### BACKGROUND

### 1. Technical Field

This document relates to devices for treating tinnitus and methods of treating tinnitus using the devices. For example, this document relates to implantable electrodes and stimulation devices for delivering electrical stimulation to the cochlear promontory, region of the cochlear promontory (including promontory surface, intraosseous, or subendosteal positions), round window niche and/or round window membrane to treat tinnitus.

### 2. Background Information

Subjective tonal tinnitus (i.e., ringing in the ear) is the phantom perception of sound when no external generating stimulus is present. Tinnitus may be unilateral, bilateral or non-localizing, and may present intermittently or continuously.

Subjective tonal tinnitus affects approximately a fourth of the US population and is a major source of disability affecting many domains of life. For some, tinnitus is merely a fleeting annoyance; however for many individuals, tinnitus may cause audiological, neurological or cognitive impairment resulting in poor attention, increased distractibility, anxiety, depression, and even suicide. Tinnitus remains the number one disability experienced by U.S. veterans. In 2011 alone, more than 10 percent of all veteran disability claims were due to tinnitus, making it a top research priority of the U.S. Department of Defense and the Veterans Health Administration.

Despite substantial clinical research in humans and study of animal models, the exact mechanism(s) behind tinnitus remain largely unknown. It is currently held that tinnitus likely reflects inadequate or maladaptive reorganization within the central nervous system following a peripheral auditory system injury. The theory of cochlear deafferentation as a cause for tinnitus parallels phantom limb pain, where cortical maladaptation develops in response to loss of sensory input.

Currently, there are no FDA approved pharmacological therapies or surgical devices available for the treatment of tinnitus. Current treatment methods largely focus on counseling, cognitive behavioral therapy, masking, and sound therapy. Such strategies may help render tinnitus more tolerable, but such strategies do not abolish the symptom or reverse the underlying pathophysiological process.

WO2017/213978 A1 describes that electrical stimulation devices can be used to treat tinnitus. For example, tinnitus can be treated using implantable electrodes and stimulation devices for delivering electrical stimulation to a patient's cochlear region. Cochlear surface electrode(s), endosteal electrode(s), subendosteal electrode(s), intraosseous electrode(s), or short intracochlear electrode(s) (or a combination thereof), connected to existing or modified cochlear implant receiver/stimulator technology, can provide a successful model for long-term treatment of tinnitus in a large number of patients. In some cases, patients can simply turn on the tinnitus implant when experiencing troublesome tinnitus and gain instant relief.

### SUMMARY

The invention is defined by the features of the independent claim 1. Preferred embodiments thereof are defined by the sub-features of the dependent claims.

This document describes devices for treating tinnitus and non-claimed methods of treating tinnitus using the devices. For example, this document describes implantable electrodes and stimulation devices for delivering electrical stimulation to the cochlear promontory to treat tinnitus. In some embodiments, the implantable electrodes and stimulation devices can be used to deliver electrical stimulation to one or more other regions including, but not limited to, the otic capsule, cochlea bone, cochlear region, vestibular region (e.g., vestibule or semicircular canals), the vestibulocochlear nerve, or the brainstem.

In one aspect, this disclosure is related to an implantable system for delivering electrical pulse stimuli to a patient's cochlear region. Such an implantable system includes: (i) a stimulator device configured to generate the electrical pulse stimuli; (ii) a lead comprising an elongate insulated electrical conductor and defining a longitudinal axis, the electrical conductor in electrical communication with the stimulator device to conduct the electrical pulse stimuli; and (iii) a single electrode disposed at a distal end of the lead and in electrical communication with the electrical conductor to deliver the electrical pulse stimuli to the patient's cochlear region. The single electrode is spaced apart from the longitudinal axis.

Such an implantable system can optionally include one or more of the following features. The single electrode may be spherical and define a center. The center of the single electrode may be spaced apart from the longitudinal axis by at least 0.4 mm. The center of the single electrode may be spaced apart from the longitudinal axis by at least 1.0 mm. The electrical conductor may include one or more bends that cause the single electrode to be spaced apart from the longitudinal axis. The lead may be a malleable member that retains a shape after being bent into the shape. In some embodiments, at least a portion of the electrical conductor extends along a helical path. The lead may include a textured portion configured to enhance cohesion of the lead with an adhesive. The system may also include a receiver coupled to the stimulator device and comprising an electrical coil configured for inductively communicating with an external device through a scalp of the patient.

In another aspect, this disclosure is directed to another implantable system for delivering electrical pulse stimuli to a patient's cochlear region. The system includes: (a) a stimulator device configured to generate the electrical pulse stimuli; (b) a lead comprising an elongate insulated electrical conductor and defining a longitudinal axis, the electrical conductor in electrical communication with the stimulator device to conduct the electrical pulse stimuli, the lead including a textured portion configured to enhance cohesion of the lead with an adhesive; and (c) a single electrode disposed at a distal end of the lead and in electrical communication with the electrical conductor to deliver the electrical pulse stimuli to the patient's cochlear region.

Such an implantable system for delivering electrical pulse stimuli to a patient's cochlear region may optionally include one or more of the following features. The system may also include a receiver coupled to the stimulator device and comprising an electrical coil configured for inductively communicating with an external device through a scalp of the patient. The textured portion may be part of an outer insulative layer over the elongate insulated electrical conductor. A portion of the electrode may be insulated by an outer insulative layer. The single electrode may be spherical and define a center. The center of the single electrode may be spaced apart from the longitudinal axis by at least 0.4 mm. The center of the single electrode may be spaced apart from the longitudinal axis by at least 1.0 mm. The electrical conductor may include one or more bends that cause the single electrode to be spaced apart from the longitudinal axis. The lead may be a malleable member that retains a shape after being bent into the shape. At least a portion of the electrical conductor may extend along a helical path.

In another aspect, this disclosure is directed to a non-claimed method of treating a tinnitus condition of a patient. The method includes: (1) drilling a recess in a cochlear promontory bone of the patient, wherein said drilling comprises creating the recess in the cochlear promontory bone without completely breaking through the cochlear promontory bone; (2) implanting an implantable system for delivering electrical pulse stimuli within the patient, wherein said implanting comprises intraosseously placing an electrode within the recess; and (3) delivering, via the electrode, electrical pulse stimuli, generated by the implantable system, to the cochlear promontory bone of the patient.

Such a method of treating a tinnitus condition of a patient may optionally include one or more of the following features. The implanting may include applying an adhesive to anchor, to the patient's anatomy, a lead comprising an elongate insulated electrical conductor of the implantable system. The lead may include a textured portion configured to enhance cohesion of the lead with the adhesive. The anatomy to which the lead is anchored may include a posterior bony ear canal of the patient. The textured portion may be part of an outer insulative layer over the elongate insulated electrical conductor. The electrode may be at the distal end of the lead. A portion of the electrode may be insulated.

Particular embodiments of the subject matter described in this document can be implemented to realize one or more of the following advantages. First, electrical stimulation delivered to the region of the cochlear promontory by an intraosseous electrode attached to an implanted receiver/stimulator electronics package, can provide an effectual long-term treatment of tinnitus in many patients. Second, in some cases the efficacy of the treatment is enhanced by locating the electrode in an intraosseous recess that is drilled in the surface of the cochlear promontory during the implant procedure. Third, in some embodiments the electrode lead has an offset tip portion that facilitates visibility and placement accuracy of the electrode during the implant procedure. Fourth, in particular embodiments the electrode lead includes a portion that is structured to enhance its affinity for cohering with an adhesive. Fifth, the electrode and stimulator systems described herein are either partially implantable or totally implantable and essentially imperceptible after implantation. Sixth, the stimulator systems described herein are configured to communicate wirelessly through the scalp of the patient with an external programmer device. Seventh, the systems described herein can treat tinnitus while only being activated for a portion of the time. For example, in some patients tinnitus will be substantially relieved with a treatment period of only about 30 minutes per day.

Unless otherwise defined, all technical and scientific terms used herein have the same meaning as commonly understood by one of ordinary skill in the art to which this invention pertains. Although methods and materials similar or equivalent to those described herein can be used to practice the invention, suitable methods and materials are described herein. All publications, patent applications, patents, and other references mentioned herein are incorporated by reference in their entirety. In case of conflict, the present specification, including definitions, will control. In addition, the materials, methods, and examples are illustrative only and not intended to be limiting.

The details of one or more embodiments of the invention are set forth in the accompanying drawings and the description herein. Other features, objects, and advantages of the invention will be apparent from the description and drawings, and from the claims.

### DESCRIPTION OF THE DRAWINGS

FIG. 1 is an illustration of an example implantable receiver/stimulator device in accordance with some embodiments.
FIG. 2 depicts a distal end portion of an example electrode device in accordance with some embodiments provided herein.
FIG. 3 depicts an example implant site for the electrode devices described herein.
FIG. 4 depicts a distal end portion of an example drill device that can be used to create a recess in bony tissue for receiving electrodes described herein.
FIG. 5 depicts the example implant site of FIG. 3 with a recess now created in the cochlear promontory.
FIG. 6 depicts a cross-sectional view of the cochlear promontory of FIG. 5.
FIG. 7 depicts the cross-sectional view of the cochlear promontory as in FIG. 6, with the addition of an electrode positioned in the recess.
FIG. 8 depicts the example implant site of FIG. 3 with an electrode positioned in the recess in the cochlear promontory.
FIG. 9 depicts the example implant site of FIG. 3 with the electrode positioned in the recess in the cochlear promontory and the electrode lead secured to the posterior bony ear canal.

Like reference numbers represent corresponding parts throughout.

### DETAILED DESCRIPTION

This document describes devices for treating tinnitus and non-claimed methods of treating tinnitus using the devices. For example, this document describes implantable intraosseous electrodes and stimulation devices for delivering electrical stimulation to the cochlear promontory to treat tinnitus. In some embodiments, the implantable electrodes and stimulation devices can also be used to deliver electrical stimulation to one or more other regions including, but not limited to, the otic capsule, cochlea bone, cochlear region, vestibular region (e.g., vestibule or semicircular canals), the vestibulocochlear nerve, or the brainstem.

In some cases, patients can treat tinnitus by self-activating the implantable intraosseous electrodes and stimulation devices described herein, for a relatively short period of time each day. For example, in some patients tinnitus will be substantially relieved with a treatment period of only about 30 minutes per day. In some cases, patients can simply turn on the tinnitus implant when experiencing troublesome tinnitus and gain relief. With increasing use, it is likely many patients will enjoy lasting tinnitus suppression, hours and even days after the device is turned off (i.e., residual inhibition).

Using intraosseous, surface, endosteal, subendosteal, or short intracochlear electrodes (or a combination thereof), customized monopolar or bipolar stimulation can be performed to target specific patterns and frequencies of tinnitus. Endosteal and/or intraosseous electrodes in the cochlear promontory can place the electrical stimulation in closer proximity to the modiolus (the conical central axis of the cochlea) without risking sensorineural hearing loss. In some cases, using a surface grid of electrodes may have the advantage of improved cochlear coverage. A short intracochlear electrode offers a direct method of cochlear stimulation. Devices and methods for each of the aforementioned treatment modalities are within the scope of this disclosure.

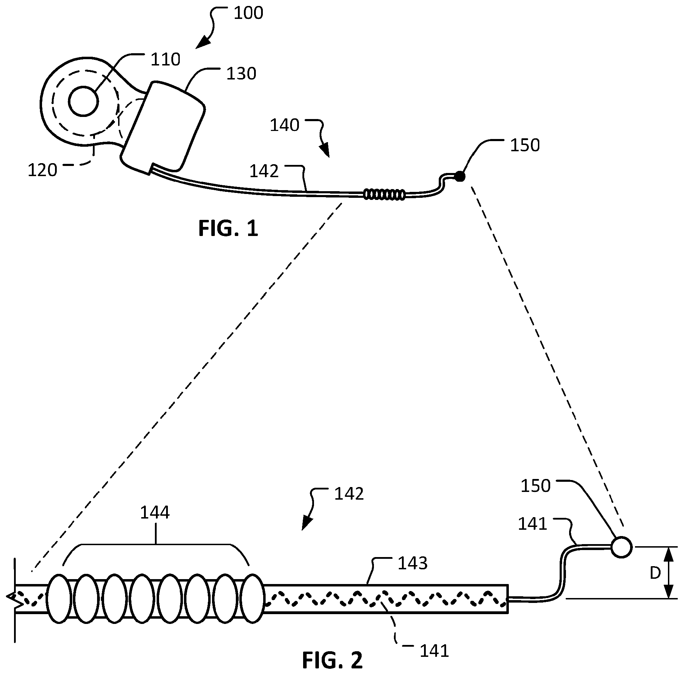

Referring to FIG. 1, an implantable receiver/stimulator device 100 can be used in conjunction with the various types of electrode devices described herein. In the depicted embodiment, an example electrode lead 140 is coupled with the implantable receiver/stimulator device 100.

In some embodiments, receiver/stimulator device 100 can be functionally akin to an implantable receiver/stimulator device used for cochlear implant electrical stimulation. Accordingly, receiver/stimulator device 100 is implanted under the post-auricular scalp and the lead wire(s) (e.g., electrode lead 140) can travel through the mastoid and facial recess to the target electrode location(s). With this system, electrical stimulation can be delivered continuously or intermittently. Further, treatment parameters can be tailored to individual patient needs according to an optimally programmed schedule, or can be administered on-demand by the patient.

In some cases, for treating tinnitus, the target electrode location may be the cochlear promontory (e.g., endosteally and, or intraosseously), bony cochlea, or otic capsule. In some cases, for treating balance disorders, the target electrode location may be the bony labyrinth (e.g., surface, intraosseous, or intra-labyrinthine) including the semicircular canals and vestibule. For example, surface, intraosseous, and intra-labyrinthine electrodes can be placed in the region of the semicircular canals and vestibule to stimulate labyrinthine function. Electrical stimulation of this organ may be used to rehabilitate vestibular hypofunction or treat ongoing or recurrent vestibular diseases, such as Meniere's disease.

In the depicted embodiment, the implantable receiver/stimulator device 100 includes a magnet 110, a receiver coil 120, and a stimulator 130. Stimulator 130 controls the operations of receiver/stimulator device 100 and is the source of the electrical stimuli (with the energy from an on-board battery). In some embodiments, the on-board battery can be inductively recharged by an external battery charger device via the receiver coil 120.

An external device (not shown) can be used to wirelessly communicate (through the patient's scalp) with the implanted receiver/stimulator device 100. Such an external device can function to activate, program, power, control, and/or otherwise interact with receiver/stimulator device 100 (e.g., to get impedance readings from the implanted receiver/stimulator device 100 to determine whether the electrode(s) is/are properly positioned).

In some cases, receiver/stimulator device 100 can be programmed to generate a particular pulse width, current amplitude, stimulus rate, stimulation mode, and the like. Magnet 110 can be used to magnetically couple and align receiver/stimulator device 100 with such an external device. Receiver coil 120 is used to communicate wirelessly with such an external device. It should be understood that the depicted embodiment of receiver/stimulator device 100 provides just one non-limiting example of the types of implantable receiver/stimulator devices that can be used in conjunction with the various types of electrode devices provided herein.

The electrode lead 140 includes an insulated lead wire 142 and an electrode 150 disposed at a distal end of the lead wire 142. The insulated lead wire 142 conducts the electrical stimuli to electrode 150. In some embodiments, the electrode lead 140 is monopolar and the casing of the implanted receiver/stimulator device 100 can act as the ground for the electrical stimuli delivered by the electrode 150. In some embodiments, one or more separate ground leads extending from the implantable receiver/stimulator device 100 is/are included.

Electrode 150 is configured to deliver the electrical stimuli to tissue of the patient. It should be understood that while a single electrode 150 is depicted, in some embodiments two or more electrodes are included. That is, various types of electrode configurations can be used for electrode 150.

In some cases, prior to permanent placement of the electrode 150, one or more test electrodes can be temporarily placed on the patient's cochlear promontory (or cochlea region) via transtympanic placement using local anesthetic with the patient awake. An instrument set can be used to apply varying patterns and/or intensities of electrical stimulation, and the patient can convey parameters resulted in greatest tinnitus reduction. Individual instruments will vary based on the number of electrodes and the distance between electrodes. Additionally, "pitch-masking" (also referred to as frequency matching) and CT imaging may assist in determining optimal positioning of the electrode 150.

Referring to **FIG. 2****,** here a distal end portion of the lead wire 140 is shown in an enlarged view. The lead wire includes the insulated lead wire 142 and the electrode 150 disposed at a distal end of the lead wire 142. The lead wire 140, in conjunction with the receiver/stimulator device 100 described above, can be used to deliver electrical pulses, e.g., to a patient's cochlear promontory, or other areas in a patient's cochlear region, to treat tinnitus.

The example lead wire 142 includes an electrical conductor 141 that is encased within a primary electrically insulative layer 143 (e.g., made of silicone or any other suitable biocompatible insulative material). The electrode 150 is attached to a distal end of the electrical conductor 141. The lead wire 142 also includes a textured region 144. As described further below, the textured region 144 can provide physical structural features to enhance the affinity of the lead wire 142 for cohering with an adhesive to anchor the lead wire 140 to the patient's tissue. Such physical structural features can include, but are not limited to, surfacing texturing, irregular surfaces with one or more peaks and/or valleys, knurling, indentations, and the like, and combinations thereof.

In some cases, the electrical conductor 141 has a diameter of 50 µm, 75 µm, 100 µm, or 125 µm, without limitation. The electrical conductor 141 can be made of any suitable material. In one example, the electrical conductor 141 is made of 90% platinum and 10% iridium. In some embodiments, the electrical conductor 141 can be configured in a helical configuration within the primary electrically insulative layer 143. Such a helical configuration can facilitate desired lateral flexibility and bending compliance properties of the lead wire 140.

The electrical conductor 141 emerges from a distal end of the primary insulative layer 143 and extends toward the electrode 150. In some embodiments, the portion of the electrical conductor 141 extending between the distal end of the primary insulative layer 143 and the electrode 150 is electrically insulated with an outer layer of electrically insulative material (e.g., silicone or another suitable insulative material). In some cases, the insulative layer 143 extends to cover and insulate the electrical conductor 141 entirely. In some cases, the insulative material can extend to cover a portion of the electrode 150, such as a proximal portion (e.g., proximal hemispherical portion) of the electrode 150. In some embodiments, that portion of the electrical conductor 141 is uninsulated.

As shown, in some embodiments the portion of the electrical conductor 141 extending between the distal end of the primary insulative layer 143 and the electrode 150 is configured so that the center of the electrode 150 is offset from the longitudinal axis of the lead wire 142 by a distance "D." The offset distance D provides for enhanced visibility of the electrode 150 during the implant procedure. In some embodiments, the offset distance D can be in a range between 0.0 to 0.5 mm, between 0.2 mm to 0.7 mm, between 0.4 mm to 0.9 mm, between 0.6 mm to 1.2 mm, or between 1.0 mm to 2.0 mm, or more than 2.0 mm, without limitation. In some embodiments, the offset distance D can be at least 0.2 mm, at least 0.4 mm, at least 0.6 mm, at least 0.8 mm, at least 1.0 mm, at least 1.2 mm, or at least 1.4 mm.

In the depicted embodiment, the portion of the electrical conductor 141 extending between the distal end of the primary insulative layer 143 and the electrode 150 is configured with two 90° bends to create the offset distance D. In some embodiments, other suitable bend configuration (e.g., two 45° bends, curves, etc.) can be used.

The offset of the portion of the electrical conductor 141 extending between the distal end of the primary insulative layer 143 and the electrode 150 also provides some springiness by which, if desired, a small preload force can be applied to bias the electrode 150 into contact with its mating surface. Alternatively, or additionally, in some embodiments the portion of the electrical conductor 141 extending between the distal end of the primary insulative layer 143 and the electrode 150 can comprise or consist of a shape-memory material (e.g., Nitinol, etc.). When heated, such a shape-memory material can deform to a shape that provides a small preload force to bias the electrode 150 into contact with its mating surface. For example, in some embodiments the pre-heated shape of the portion of the electrical conductor 141 extending between the distal end of the primary insulative layer 143 and the electrode 150 can be bent as depicted in FIG. 2, and when heated one or more of the bends can tend to straighten and thereby provide a small preload force to bias the electrode 150 into contact with its mating surface.

In some embodiments, the textured region 144 can be a molded portion of the primary insulative layer 143 that creates multiple ridges and recesses in the outer diameter of the otherwise cylindrical primary insulative layer 143. In some embodiments, the texture region 144 is about 9 mm long and ends about 6 mm from the distal end of the primary insulative layer 143. The textured region 144, along with an adhesive (e.g., otologic bone cement and the like), can be used to anchor the lead wire 142 to the patient's tissue at the target site, and to provide migration resistance so that the electrode 150 stays positioned relative to the patient's anatomy as desired. One example of a suitable bone cement is "FUSE Glass Ionomer Cement" or "ProCem Otologic Cement" from Grace Medical, Memphis, Tennessee, USA. In some cases, one or more mechanical anchors such as a screw, clip, helix, suture, or barbed member can be used (additionally or alternatively) to anchor the lead wire 142 to tissue. In some embodiments, the lead wire 142 can include one or more fenestrations that can receive adhesive and/or a mechanical anchor.

The electrode 150 is spherical in the depicted example. In some embodiments, the electrode 150 can be cylindrical, conical, frustoconical, a polyhedron, and combinations thereof. The diameter of the spherical electrode 150 can be in a range between 0.0 to 0.4 mm, between 0.1 mm to 0.5 mm, between 0.2 mm to 0.6 mm, between 0.3 mm to 0.7 mm, or between 0.4 mm to 0.9 mm, or between 0.6 mm to 1.2 mm, or between 0.8 mm to 1.6 mm, or between 1.4 mm to 2.0 mm, or more than 2.0 mm, without limitation. In some embodiments, the electrode 150 is created by flaming the electrical conductor 141, or by any other suitable method (e.g., laser welding, using an adhesive, etc.).

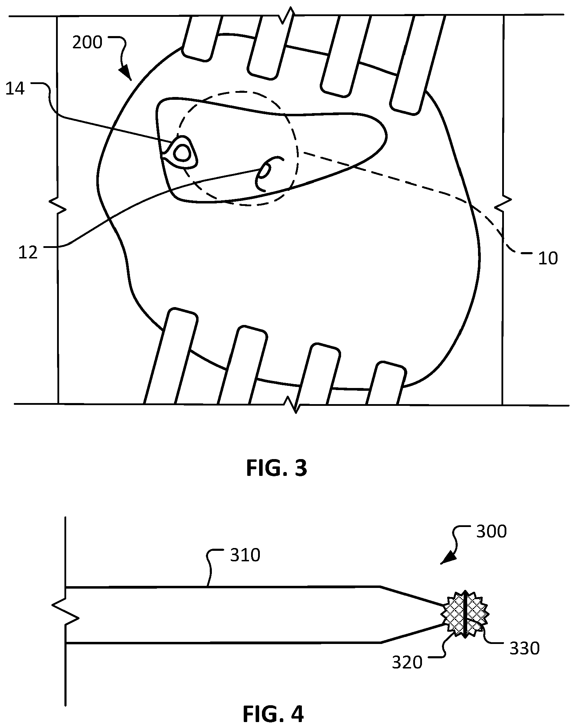

Referring to FIG. 3, a surgical site 200 of a patient is depicted. The surgical site 200 is made by a postauricular (behind the ear, such as behind the right ear in the depicted example) surgical incision that extends through the skin and subcutaneous tissue of the patient. The promontory is then exposed through a posterior tympanotomy (facial recess). Portions of the cochlear promontory 10 are thereby exposed for access. To orient the reader/viewer to the location of the surgical site 200, some other anatomical landmarks are shown, including the cochlear round window 12, the stapes 14, and the pharyngotympanic (auditory) tube 16.

Referring also to **FIG. 4****,** in some embodiments an example micro-drill instrument 300 can be used with a rotary driver to create a recess in the cochlear promontory 10 that will receive the electrode 150 (FIG. 2). Placement of the electrode 150 intraosseously in the cochlear promontory is, in some cases, preferred to a purely surface contact electrode because an intraosseous design confers a more stable electrode bed to reduce electrode migration or displacement; increases the surface area of electrode-bone contact; reduces the probability of untoward current spread during stimulation that might result in discomfort or facial nerve stimulation; and reduces stimulation threshold requirements thereby enhancing battery life and mitigating aberrant electrical stimulation.

The example micro-drill instrument 300 includes a shank 310 and a working portion 320 at a distal end of the shank 310. Working portion 320 includes cutting edges (course fluted burrs or more smooth diamond burrs) that can remove tissue such as bone tissue to create a recess or hole (e.g., such as a blind hole or through hole) in the target tissue layer (e.g., anywhere on the cochlear promontory 10 including near or at the oval window, near or at the round window 12, etc.). In the depicted embodiment, the working portion 320 is spherical to correspond to the spherical electrode 150 (FIG. 2). That is, the working portion 320 is sized and shaped to create a recess that will be sized and shaped to receive the spherical electrode 150. For example, the working portion 320 can have a diameter in a range between 0.0 to 0.4 mm, between 0.1 mm to 0.5 mm, between 0.2 mm to 0.6 mm, between 0.3 mm to 0.7 mm, or between 0.4 mm to 0.9 mm, or between 0.6 mm to 1.2 mm, or between 0.8 mm to 1.6 mm, or between 1.4 mm to 2.0 mm, or more than 2.0 mm, without limitation.

The micro-drill instrument 300 can include a depth limiter or indicator. For example, in the depicted embodiment the working portion 320 includes a circumferential marker 330 that can be used a visual indication of the depth to which the working portion 320 has penetrated into a substance such as the cochlear promontory 10. The depth marker 330 can advantageously prevent the recess created in the cochlear promontory 10 from becoming a through-hole (i.e., from penetrating completely through the opposite side of the cochlear promontory 10). In general, the target depth for the recess to be created in the cochlear promontory 10 is between about 1/2 and 2/3 of the thickness of the wall of the cochlear promontory 10.

Other types of depth limiters or indicators are also envisioned. For example, an annular ring can be included on the working portion 320 instead of, or in addition to, the circumferential marker 330. In some cases, a side-arm stopper extending along the side the micro-drill instrument 300 can be attached to the rotary driver. Patient populations naturally have differing anatomical features (such as promontory thicknesses and the like). Accordingly, a variety of differently sized drill instruments 300 can be available so as to suit an individual patient's anatomy and/or electrode size.

In most cases, the most suitable micro-drill instruments 300 and/or electrode device for a particular patient can be determined in advance of the implant procedure. For example, in some cases a patient can undergo a pre-operative imaging procedure, such as a computerized tomography (CT) scan, to determine the patient's anatomical features such as, but not limited to, promontory thickness. Based on the inventor's investigations, minimal promontory thickness is about 0.4-0.5 mm and maximal promontory thickness is about 2.0-2.2 mm. Thus, a desirable hole depth (and intraosseous electrode length) can be about 0.3 mm to about 0.7 mm, or about 0.5 mm to about 0.9 mm, or about 0.7 mm to about 1.1 mm, or about 0.9 mm to about 1.3 mm, or about 1.1 mm to about 1.5 mm, or about 1.3 mm to about 1.7 mm, or about 1.5 mm to about 1.9 mm, or about 1.7 mm to about 2.1 mm, and/or anywhere within such ranges. In some cases, a set of multiple drill instruments 300 will be made available in 0.2 mm depth increments, or 0.1 mm depth increments.

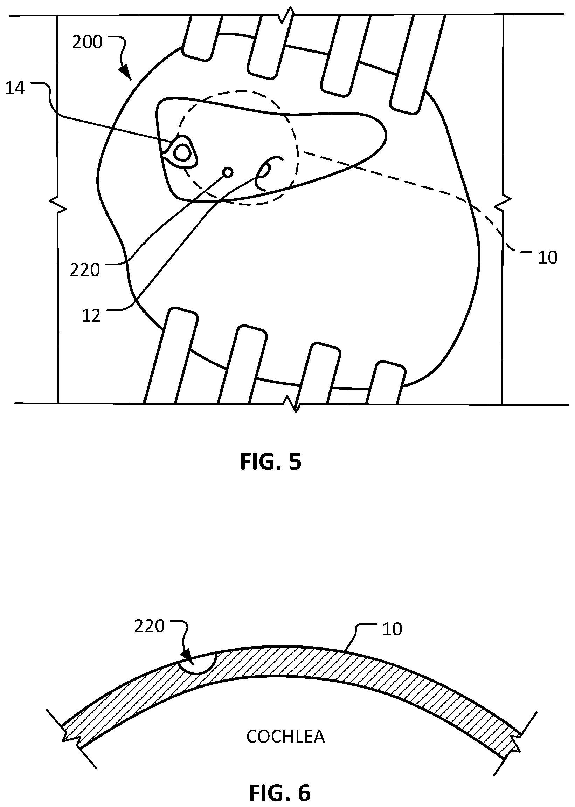

Referring now to **FIGs. 5 and 6****,** a recess 220 in the cochlear promontory 10 has been created in preparation for receiving the intraosseous electrode 150 (FIG. 2). The recess 220 can be created using the drill instrument 300, or any other suitable instrument/technique. In general, the target depth for the recess to be created in the cochlear promontory 10 is between about 1/2 and 2/3 of the thickness of the wall of the cochlear promontory 10 (i.e., without breaking through the entire thickness of the cochlear promontory 10).

The recess 220 can be created anywhere on the cochlear promontory 10. For example, in the depicted embodiment the recess 220 is near to the round window 12. As an alternative to placing the electrode 150 in the recess 220 as described herein, in some embodiments a plug containing an electrode can be placed into the round window 12, for example.

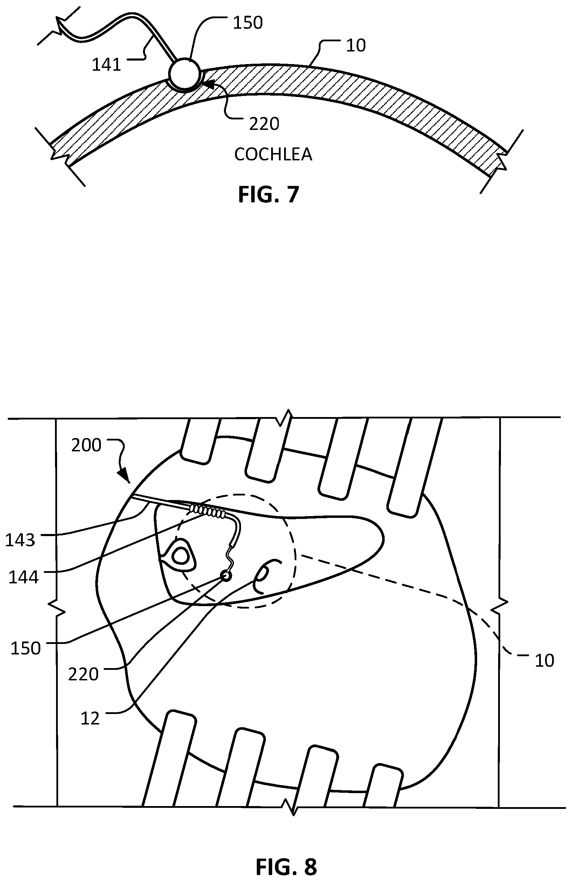

Referring now to **FIGs. 7 and 8****,** the implantable receiver/stimulator device 100 (refer to FIG. 1) and the electrode lead 140 are shown as implanted at/via the surgical site 200. Accordingly, receiver/stimulator device 100 is implanted under the post-auricular scalp (not visible) and the electrode lead 140 is extending therefrom through the mastoid and facial recess such that the electrode 150 is positioned in the recess 220 in the cochlear promontory 10.

The electrode lead 140 is installed relative to the anatomy so that there is a slight pressure exerted by the electrode 150 to the recess 220. In some embodiments, the electrode lead 140 is suitably shapeable/malleable to allow for the electrode lead 140 to be shaped and configured as needed for the implantation procedure. In some such embodiments, the electrode lead 140 will tend to retain the shape to which it is configured.

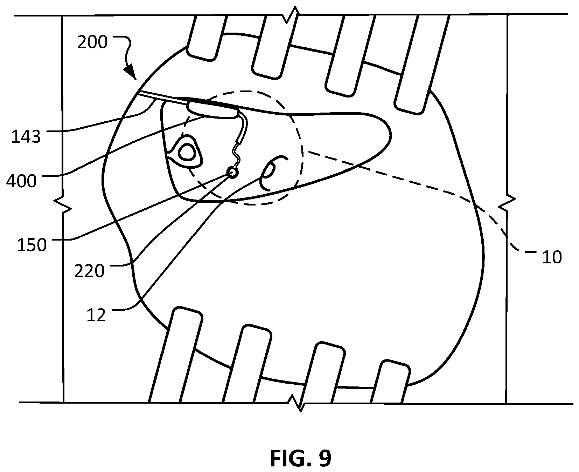

Referring to **FIG. 9****,** in some embodiments an adhesive 400 (e.g., otologic bone cement, tissue glue, etc.) can be used to anchor the electrode lead 140 to the patient's anatomy for migration resistance. For example, in the depicted implantation a bone cement 400 is adhering the electrode lead 140 (as facilitated by the textured region 144, obscured from view here; refer to FIG. 8) to the patient's posterior bony ear canal. This completes the implantation process of the implantable receiver/stimulator device 100 (refer to FIG. 1) and the electrode lead 140 which can thereafter be used to deliver electrical stimuli intraosseously to the cochlear promontory 10 to treat tinnitus.

### ADDITIONAL INFORMATION, DESIGN VARIATIONS AND EMBODIMENTS

The systems described herein for treating tinnitus by delivering stimuli intraosseously to the cochlear promontory can be adjusted to deliver a range of various stimulation parameters (amplitude, pulse width, etc.). For example, in some cases stimulation pulses can be biphasic or triphasic pulses charge balanced delivered in a monopolar configuration. Parameters can be set up based on subjective patient feedback. Upper stimulation limits can be applied from the existing limits used in Cochlear Implants. In some cases, pulse duration can be limited to a maximum of 200 microseconds, and charge per phase can be limited to 282.8 nC. In some cases, the charge per phase could be chosen higher in the tinnitus implants described herein, as the geometric surface area of the ball contact of the tinnitus implant electrode (e.g., about 0.48 mm^2) is significantly larger than the geometric surface area per cochlear implant channel (e.g., about 0.14 mm^2).

The surgical procedure for implanting the systems described herein for treating tinnitus by delivering stimuli intraosseously to the cochlear promontory is further described as follows. Following induction of general endotracheal anesthesia, the subject is placed in a supine position with the head gently turned away. Bipolar orbicularis oculi and orbicularis oris facial nerve monitoring electrodes are placed for continuous intraoperative facial nerve electromyographic monitoring. The subject is then prepped and draped in the usual fashion for otologic surgery. A postauricular incision is marked and infiltrated with lidocaine with epinephrine 1:1000. The planned position of the device is then marked to facilitate accurate placement later in the case.

A postauricular incision is then made through skin and subcutaneous tissue and elevated forward in a loose areolar plane. A separate staggered musculoperiosteal incision is then made to the mastoid cortex and elevated forward in a subperiosteal plane. A self-retaining retractor is then placed.

Next, using an operative microscope, a cortical mastoidectomy with antrotomy is performed using a combination of cutting and diamond drill bits and continuous irrigation. Care is taken to avoid uncovering the temporal dura or sigmoid sinus. A standard facial recess (posterior tympanotomy) is made, preserving the chorda tympani and facial nerve. The position on the promontory for electrode placement is marked. Using an otologic mini-drill (e.g., with a 0.5mm drill bit), a small well is created on the cochlear promontory surface to accommodate the intraosseous promontory electrode. Care is taken to not breach the endosteum of the cochlea or enter the cochlear lumen.

Next, a tight subperiosteal pocket is created under the temporal scalp and temporalis muscle to fixate the internal device. An electrode channel is then drilled to accommodate the electrode lead. The surgical field is then copiously irrigated with antibiotic solution, meticulous hemostasis is obtained, and the surgeon's gloves are changed to maximize field sterility.

The device is then brought into the field and monopolar cautery is removed from the field. The device is placed in a tight subperiosteal pocked and the electrode contact is positioned within the cochlear promontory well. After ensuring good bone contact via direct microscopic visualization and device impedance testing, the electrode is secured to the posterior bony ear canal (e.g., using otologic bone cement). The bone cement is left undisturbed for 5 minutes to cure and harden. The redundant portion of the electrode is then coiled in the mastoid. Adequate electrode contact between the electrode and promontory well is once again confirmed visually and via impedance testing.

The incision is then closed in anatomical layers using single interrupted suture and a standard otologic headwrap is applied. The subject is then awakened, extubated, and transferred to the post-anesthesia care unit for recovery. Following surgery, the subject is examined by the surgeon to ensure they have not experienced any adverse events related to surgery. Once standard outpatient discharge criteria have been met, the subject is discharged from the hospital.

Experiments have been performed to confirm the feasibility of the systems described herein for treating tinnitus. Twenty-five subjects enrolled in the study, although three withdrew before undergoing promontory stimulation. The mean age at enrollment for the remaining 22 subjects was 59.3 years (SD 7.7) and included 14 (64%) men and 8 (36%) women.

Each patient received three sessions of in-office promontory stimulation using biphasic charge balanced pulses. Following successful transtympanic placement of an insulated monopolar stimulation probe, a calibration session to assess optimal stimulation parameters for the therapeutic session was carried out using an output of 0 to 1000 µA for pulse frequencies 100 Hz, 800 Hz and 1600 Hz. For each stimulus frequency, current levels were gradually up-titrated to determine the following perceptual parameters: 1) detection threshold defined as the first detection of the electrical stimulus (tactile or auditory) as reported by the subject; 2) maximum comfort threshold defined as the level first causing discomfort as reported by the subject.

The subject then received 10 minutes of stimulation at 80% of the maximum comfort threshold for each of the predetermined pulse frequencies. Each subject underwent this stimulation cycle in three separate sessions, each spaced one week apart.

Efficacy of stimulation was assessed by comparing baseline scores to post-stimulation scores from three validated tinnitus questionnaires: TFI, THI, and Tinnitus VAS. Safety of stimulation was primarily assessed by comparing baseline hearing thresholds via standard behavioral audiometry to post-stimulation thresholds for the conventional frequencies 0.25 to 8 kHz, including interoctaves.

All 22 subjects had clinically significant improvement in THI score defined as a change of at least 7 points; 20 had clinically significant improvement in TFI score defined as a change of at least 13 points; and 17 had clinically significant improvement in Tinnitus VAS score defined as a change of at least 20 points. In total, 17 (77%) subjects had clinically significant improvement in all three scores, 20 (91%) had improvement in two of the three scores, and 22 (100%) had improvement in one of the three scores.

In some cases, patients experience tinnitus in just one ear. In other cases, patients experience tinnitus in both ears. A single system described herein for treating tinnitus by delivering stimuli intraosseously to the cochlear promontory can be implanted to treat either condition (i.e., tinnitus in one ear or in both ears). Alternatively, in some cases two systems described herein for treating tinnitus by delivering stimuli intraosseously to the cochlear promontory can be implanted in a single patient to treat tinnitus in both ears.

While this specification contains many specific implementation details, these should not be construed as limitations on the scope of any invention or of what may be claimed, but rather as descriptions of features that may be specific to particular embodiments of particular inventions. Certain features that are described in this specification in the context of separate embodiments can also be implemented in combination in a single embodiment. Conversely, various features that are described in the context of a single embodiment can also be implemented in multiple embodiments separately or in any suitable subcombination. Moreover, although features may be described herein as acting in certain combinations and even initially claimed as such, one or more features from a claimed combination can in some cases be excised from the combination, and the claimed combination may be directed to a subcombination or variation of a subcombination.

Similarly, while operations are depicted in the drawings in a particular order, this should not be understood as requiring that such operations be performed in the particular order shown or in sequential order, or that all illustrated operations be performed, to achieve desirable results. In certain circumstances, multitasking and parallel processing may be advantageous. Moreover, the separation of various system modules and components in the embodiments described herein should not be understood as requiring such separation in all embodiments, and it should be understood that the described program components and systems can generally be integrated together in a single product or packaged into multiple products.

Particular embodiments of the subject matter have been described. Other embodiments are within the scope of the following claims. For example, the actions recited in the claims can be performed in a different order and still achieve desirable results. As one example, the processes depicted in the accompanying figures do not necessarily require the particular order shown, or sequential order, to achieve desirable results. In certain implementations, multitasking and parallel processing may be advantageous.

## Claims

1. An implantable system (100) for delivering electrical pulse stimuli to a patient's cochlear region, the system (100) comprising:
a stimulator device (130) configured to generate the electrical pulse stimuli;
a lead (140) comprising an elongate insulated electrical conductor (142) and defining a longitudinal axis, the electrical conductor (142) in electrical communication with the stimulator device (130) to conduct the electrical pulse stimuli, the lead (140) including a textured portion (144) configured to enhance cohesion of the lead (140) with an adhesive; and
a single electrode (150) disposed at a distal end of the lead (140) and in electrical communication with the electrical conductor (142) to deliver the electrical pulse stimuli to the patient's cochlear region.

2. The system of claim 1, further comprising a receiver coupled to the stimulator device and comprising an electrical coil configured for inductively communicating with an external device through a scalp of the patient.

3. The system of any one of claims 1 and 2, wherein the textured portion is part of an outer insulative layer over the elongate insulated electrical conductor.

4. The system of any one of claims 1 through 3, wherein an insulated portion of the electrode is insulated by an outer insulative layer.

5. The system of claim 4, wherein the insulated portion of the electrode includes a proximal portion of the electrode and a distal portion of the electrode is uninsulated.

6. The system of any one of claims 1 through 5, wherein the lead comprises a shape-memory material that is configured to change shape in response to being heated.

7. The system of claim 6, wherein the shape-memory material comprises Nitinol.

8. An implantable system (100) of claim 1 for use in a surgical method of treating a tinnitus condition of a patient, the method comprising:
drilling a recess (220) in a cochlear promontory bone (10) of the patient, wherein said drilling comprises creating the recess (220) in the cochlear promontory bone (10) without completely breaking through the cochlear promontory bone (220);
implanting said implantable system (100) for delivering electrical pulse stimuli within the patient, wherein said implanting comprises intraosseously placing an electrode (150) within the recess (220); and
delivering, via the electrode (150), electrical pulse stimuli, generated by the implantable system (100), to the cochlear promontory bone (10) of the patient;
wherein the implanting includes applying an adhesive to anchor, to the patient's anatomy, a lead (140) comprising an elongate insulated electrical conductor (142) of the implantable system (100), and wherein the lead (140) includes a textured portion (144) configured to enhance cohesion of the lead (140) with the adhesive.

9. The system for use of claim 8, wherein the anatomy to which the lead is anchored comprises a posterior bony ear canal of the patient.

10. The system for use of claim 8, wherein the textured portion is part of an outer insulative layer over the elongate insulated electrical conductor.

11. The system for use of claim 8, wherein the electrode is at the distal end of the lead and an insulated portion of the electrode is insulated by an outer insulative layer, optionally wherein the insulated portion of the electrode includes a proximal portion of the electrode and a distal portion of the electrode is uninsulated.

12. The system for use of claim 11, wherein the intraosseously placing the electrode within the recess results in an ongoing contact pressure that is exerted by the electrode to a wall surface of the cochlear promontory bone that defines the recess, optionally wherein the electrode is connected to a distal end of a lead that provides springiness to generate the ongoing contact pressure.

13. The system for use of any one of claims 8 through 12, wherein the electrode is connected to a distal end of a lead that comprises a shape-memory material configured to change shape in response to being heated, optionally further comprising:
heating the shape-memory material, wherein the heating causes the lead to change shape so as to cause an ongoing contact pressure that is exerted by the electrode to a wall surface of the cochlear promontory bone that defines the recess.

14. The system for use of claim 13, wherein the shape-memory material comprises Nitinol.

15. The system of any one of claims 1 through 7 or the system for use of any one of claims 8 through 14, wherein the textured portion comprises physical structural features selected from an irregular surface with one or more peaks and valleys, knurling, indentations, and combinations thereof.

## Patentansprüche

1. Implantierbares System (100) zum Abgeben elektrischer Impulsreize an die Cochlea-Region eines Patienten, wobei das System (100) umfasst:
eine Stimulationsvorrichtung (130), die zum Erzeugen der elektrischen Impulsreize konfiguriert ist;
eine Leitung (140), die einen länglichen isolierten elektrischen Leiter (142) aufweist und eine Längsachse definiert, wobei der elektrische Leiter (142) in elektrischer Verbindung mit der Stimulationsvorrichtung (130) steht, um die elektrischen Impulsreize zu leiten, wobei die Leitung (140) einen strukturierten Abschnitt (144) aufweist, der ausgebildet ist, Kohäsion der Leitung (140) mit einem Klebstoff zu verbessern; und
eine einzelne Elektrode (150), die an einem distalen Ende der Leitung (140) angeordnet ist und in elektrischer Verbindung mit dem elektrischen Leiter (142) steht, um die elektrischen Impulsreize an die Cochlea-Region des Patienten abzugeben.

2. System nach Anspruch 1, das ferner einen mit der Stimulationsvorrichtung gekoppelten Empfänger aufweist, der eine elektrische Spule aufweist, die ausgebildet ist, induktiv mit einer externen Vorrichtung über die Kopfhaut des Patienten zu kommunizieren.

3. System nach Anspruch 1 oder 2, wobei der strukturierte Abschnitt Teil einer äußeren Isolierschicht über dem länglichen isolierten elektrischen Leiter ist.

4. System nach einem der Ansprüche 1 bis 3, wobei ein isolierter Abschnitt der Elektrode durch eine äußere Isolierschicht isoliert ist.

5. System nach Anspruch 4, wobei der isolierte Abschnitt der Elektrode einen proximalen Abschnitt der Elektrode aufweist und ein distaler Abschnitt der Elektrode nicht isoliert ist.

6. System nach einem der Ansprüche 1 bis 5, wobei die Leitung ein Formgedächtnismaterial aufweist, das ausgebildet ist, seine Form in Reaktion auf Erwärmung zu ändern.

7. System nach Anspruch 6, wobei das Formgedächtnismaterial Nitinol aufweist.

8. Implantierbares System (100) gemäß Anspruch 1 zur Verwendung in einem chirurgischen Verfahren zur Behandlung von Tinnitus eines Patienten, wobei das Verfahren umfasst:
Bohren einer Aussparung (220) in einen Cochlea-Promontoriumsknochen (10) des Patienten, wobei das Bohren Erzeugen der Aussparung (220) in dem Cochlea-Promontoriumsknochen (10) beinhaltet, ohne den Cochlea-Promontoriumsknochen (220) vollständig zu durchbrechen;
Implantieren des implantierbaren Systems (100) zum Abgeben elektrischer Impulsreize in den Patienten, wobei das Implantieren intraossäres Platzieren einer Elektrode (150) in der Aussparung (220) beinhaltet; und
Zuführen, über die Elektrode (150), elektrischer Impulsreize, die durch das implantierbare System (100) erzeugt werden, zu dem Cochlea-Promontoriumsknochen (10) des Patienten;
wobei das Implantieren Aufbringen eines Klebstoffs zum Verankern einer Leitung (140), die einen länglichen isolierten elektrischen Leiter (142) des implantierbaren Systems (100) aufweist, an der Anatomie des Patienten umfasst, und wobei die Leitung (140) einen strukturierten Abschnitt (144) aufweist, der ausgebildet ist, Kohäsion der Leitung (140) mit dem Klebstoff zu verbessern.

9. System zur Verwendung nach Anspruch 8, wobei die Anatomie, an der die Leitung verankert wird, einen hinteren knöchernen Gehörgang des Patienten beinhaltet.

10. System zur Verwendung nach Anspruch 8, wobei der strukturierte Abschnitt Teil einer äußeren Isolierschicht über dem länglichen isolierten elektrischen Leiter ist.

11. System zur Verwendung nach Anspruch 8, wobei sich die Elektrode an dem distalen Ende der Leitung befindet und ein isolierter Abschnitt der Elektrode durch eine äußere Isolierschicht isoliert ist, wobei optional der isolierte Abschnitt der Elektrode einen proximalen Abschnitt der Elektrode enthält und ein distaler Abschnitt der Elektrode nicht isoliert ist.

12. System zur Verwendung nach Anspruch 11, wobei das intraossäre Anordnen der Elektrode in der Aussparung zu einem anhaltenden Kontaktdruck führt, der von der Elektrode auf eine Wandfläche des Cochlea-Promontoriumsknochens ausgeübt wird, der die Aussparung begrenzt, wobei optional die Elektrode mit einem distalen Ende einer Leitung verbunden ist, die eine Federkraft bereitstellt, um den anhaltenden Kontaktdruck zu erzeugen.

13. System zur Verwendung nach einem der Ansprüche 8 bis 12, wobei die Elektrode mit einem distalen Ende einer Leitung verbunden ist, die ein Formgedächtnismaterial aufweist, das ausgebildet ist, seine Form in Reaktion auf Erwärmung zu ändern, optional ferner umfassend:
Erwärmen des Formgedächtnismaterials, wobei das Erwärmen bewirkt, dass die Leitung ihre Form ändert, um einen anhaltenden Kontaktdruck zu erzeugen, der von der Elektrode auf eine Wandfläche des Cochlea-Promontoriumsknochens ausgeübt wird, der die Aussparung begrenzt.

14. System zur Verwendung nach Anspruch 13, wobei das Formgedächtnismaterial Nitinol aufweist.

15. System nach einem der Ansprüche 1 bis 7 oder das System zur Verwendung nach einem der Ansprüche 8 bis 14, wobei der strukturierte Abschnitt physikalische Strukturmerkmale aufweist, die aus einer unregelmäßigen Oberfläche mit einer oder mehreren Erhebungen und Tälern, Rändelungen, Vertiefungen und Kombinationen davon ausgewählt sind.

## Revendications

1. Système implantable (100) d'administration de stimuli d'impulsion électrique à une région cochléaire d'un patient, le système (100) comprenant :
un dispositif stimulateur (130) configuré pour générer les stimuli d'impulsion électrique ;
un fil (140) comprenant un conducteur électrique isolé allongé (142) et définissant un axe longitudinal, le conducteur électrique (142) en communication électrique avec le dispositif stimulateur (130) pour conduire les stimuli d'impulsion électrique, le fil (140) incluant une portion texturée (144) configurée pour améliorer une cohésion du fil (140) avec un adhésif ; et
une électrode unique (150) disposée au niveau d'une extrémité distale du fil (140) et en communication électrique avec le conducteur électrique (142) pour administrer les stimuli d'impulsion électrique à la région cochléaire du patient.

2. Système selon la revendication 1, comprenant en outre un récepteur accouplé au dispositif stimulateur et comprenant une bobine électrique configurée pour communiquer de manière inductive avec un dispositif externe à travers le cuir chevelu du patient.

3. Système selon l'une quelconque des revendications 1 et 2, dans lequel la portion texturée fait partie d'une couche isolante externe sur le conducteur électrique isolé allongé.

4. Système selon l'une quelconque des revendications 1 à 3, dans lequel une portion isolée de l'électrode est isolée par une couche isolante externe.

5. Système selon la revendication 4, dans lequel la portion isolée de l'électrode inclut une portion proximale de l'électrode et une portion distale de l'électrode n'est pas isolée.

6. Système selon l'une quelconque des revendications 1 à 5, dans lequel le fil comprend un matériau à mémoire de forme qui est configuré pour changer de forme en réponse à son chauffage.

7. Système selon la revendication 6, dans lequel le matériau à mémoire de forme comprend du nitinol.

8. Système implantable (100) selon la revendication 1, pour l'utilisation dans un procédé chirurgical de traitement d'acouphènes chez un patient, le procédé comprenant les étapes consistant à :
percer un renfoncement (220) dans un os promontoire de la cochlée (10) du patient, dans lequel ladite perforation comprend une création du renfoncement (220) dans l'os promontoire de la cochlée (10) sans percer entièrement l'os promontoire de la cochlée (220) ;
implanter ledit système implantable (100) pour administrer des stimuli d'impulsion électrique chez le patient, dans lequel ladite implantation comprend la mise en place de manière intra-osseuse d'une électrode (150) à l'intérieur du renfoncement (220) ; et
l'administration, via l'électrode (150), de stimuli d'impulsion électrique, générés par le système implantable (100), à l'os promontoire de la cochlée (10) du patient ;
dans lequel l'implantation inclut l'application d'un adhésif pour ancrer, au niveau de l'anatomie du patient, un fil (140) comprenant un conducteur électrique isolé allongé (142) du système implantable (100), et dans lequel le fil (140) inclut une portion texturée (144) configurée pour améliorer une cohésion du fil (140) avec l'adhésif.

9. Système pour l'utilisation selon la revendication 8, dans lequel l'anatomie à laquelle le fil est ancré comprend un canal auditif osseux postérieur du patient.

10. Système pour l'utilisation selon la revendication 8, dans lequel la portion texturée fait partie d'une couche isolante externe sur le conducteur électrique isolé allongé.

11. Système pour l'utilisation selon la revendication 8, dans lequel l'électrode se trouve au niveau de l'extrémité distale du fil et une portion isolée de l'électrode est isolée par une couche isolante externe, éventuellement dans lequel la portion isolée de l'électrode inclut une portion proximale de l'électrode et une portion distale de l'électrode n'est pas isolée.

12. Système pour l'utilisation selon la revendication 11, dans lequel la mise en place de manière intra-osseuse de l'électrode à l'intérieur du renfoncement résulte en une pression de contact continue qui est exercée par l'électrode à une surface de paroi de l'os promontoire de la cochlée qui définit le renfoncement, éventuellement dans lequel l'électrode est connectée à une extrémité distale d'un fil qui fournit de l'élasticité pour générer la pression de contact continue.

13. Système pour l'utilisation selon l'une quelconque des revendications 8 à 12, dans lequel l'électrode est connectée à une extrémité distale d'un fil qui comprend un matériau à mémoire de forme configuré pour changer de forme en réponse à son chauffage, éventuellement comprenant en outre :
le chauffage du matériau à mémoire de forme, dans lequel le chauffage amène le fil à changer de forme afin de générer une pression de contact continue qui est exercée par l'électrode à une surface de paroi de l'os promontoire de la cochlée qui définit le renfoncement.

14. Système pour l'utilisation selon la revendication 13, dans lequel le matériau à mémoire de forme comprend du nitinol.

15. Système selon l'une quelconque des revendications 1 à 7 ou système pour l'utilisation selon l'une quelconque des revendications 8 à 14, dans lequel la portion texturée comprend des caractéristiques structurales physiques sélectionnées parmi une surface irrégulière avec un ou plusieurs pics et vallées, un moletage, des dentelures, et des combinaisons de ceux-ci.
